# EUROPÄISCHE PATENTSCHRIFT

(11) **EP 1 372 520 B1**
(45) Veröffentlichungstag und Bekanntmachung des Hinweises auf die Patenterteilung: **26.03.2008**
(21) Anmeldenummer: 01274022.1
(22) Anmeldetag: 23.11.2001
(51) Int. Cl.: A61C 13/00, A61K 6/027, C04B 28/14

(54) **EXPANDIERENDES MODELLMATERIAL FÜR ZAHNTECHNISCHE ZWECKE**
EXPANDING DENTAL MODEL MATERIAL
MATERIAU EXPANSIF DESTINE A DES MODELES DENTAIRES

(30) Priorität: 26.03.2001 DE 10115820
(43) Veröffentlichungstag der Anmeldung: 02.01.2004
(73) Patentinhaber: Wieland Dental + Technik GmbH & Co. KG, 75179 Pforzheim (DE)
(72) Erfinder: LAUBERSHEIMER, Jürgen, 76307 Karlsbad (DE)
(74) Vertreter: Patentanwälte Ruff, Wilhelm, Beier, Dauster & Partner
(86) Internationale Anmeldenummer: PCT/EP2001/013640
(87) Internationale Veröffentlichungsnummer: WO 2002/076325

(56) Entgegenhaltungen:
- DE-A- 3 500 515
- GB-A- 497 945
- US-A- 4 772 436

## Beschreibung

Die Erfindung betrifft ein Modellmaterial für zahntechnische Zwecke sowie ein Verfahren zur Herstellung eines zahntechnischen Modells, insbesondere eines sogenannten zahntechnischen Arbeitsmodells.

Schon immer war Keramik oder "Porzellan" ein attraktiver Werkstoff, um Zähne mit sehr zahnähnlichem Aussehen in Form und Farbe nachzubilden. Keramik ist ein chemisch beständiger, korrosionsfester und biokompatibler Werkstoff, der zudem noch in schier unendlicher Menge in mineralischer Form verfügbar und somit preiswert ist. Aus diesem Werkstoff ist mit zahntechnischen Mitteln individueller Zahnersatz einfach und reproduzierbar herzustellen, so dass der Durchbruch des Werkstoffes "Dentalkeramik" eingetreten ist.

Um die einzige Schwäche dieses Werkstoffes, die Sprödigkeit, zu umgehen, wird zahntechnisch gefertigter Zahnersatz in der Regel schon seit langem als klassischer Werkstoff-Verbund hergestellt, z.B. als sogenannte Metallkeramik. Eine metallkeramische Krone oder Brücke besteht aus einem metallischen Gerüst bzw. Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als (Schutz-) Käppchen bezeichnet. Je nachdem, aus welchem Material bzw. aus welcher Legierung die Käppchen bestehen und je nach Herstellungsverfahren (Giessen, Galvanoforming-Verfahren, d. h. galvanische Abscheidung), können Probleme in Form von Korrosion und daraus resultierende Verfärbungen, Körperunverträglichkeiten u.a.m. entstehen. Deshalb wurden in den letzten Jahren zunehmend Systeme entwickelt, die vergleichbare Unterkonstruktionen aus keramischen Materialien herstellen und zahntechnisch weiterverarbeiten können.

Es gibt bereits mehrere funktionierende Systeme auf dem Dentalmarkt. So werden die Keramik-Käppchen beispielsweise durch manuelles Auftragen eines Schlickers auf einen Modellstumpf, anschließendem Sinterbrand sowie nachfolgender Infiltration mit Spezialglas (VITA In-Ceram) oder durch einen Pressvorgang unter Temperatureinwirkung (Empress, Fa. IVOCLAR) hergestellt. Es gibt auch Systeme, bei denen die Käppchen aus gesinterten oder vorgesinterten Keramikblöcken digital gefräst werden (DCS-System, CEREC usw.). Allen solchen sogenannten Vollkeramik-Systemen ist jedoch gemeinsam, dass sie die Passgenauigkeit metallischer Körper auf dem Restzahn, ob letztere nun gegossen sind oder durch galvanische Prozesse entstehen, in der Regel nicht erreichen. Zudem sind diese Systeme in der Anschaffung meist sehr teuer.

Die mangelnde Passgenauigkeit existierender Vollkeramik-Systeme ergibt sich hauptsächlich durch die verwendeten Formgebungsverfahren. Bei der Herstellung metallischer Käppchen wird gegossen oder galvanisiert, so dass sich das Metall in geschmolzener bzw. gelöster Form optimal der Stumpfgeometrie anpassen kann. Dagegen muss z. B. bei CADCAM-gestützten Vollkeramikverfahren nach einem digital aufgenommenen Datensatz aus festem Material spanabhebend gefräst werden. Das Scannen des Zahnstupfes und das Fräsen können aber, je nach der digitalen Auflösung der Systemkomponenten, bereits Ungenauigkeiten enthalten.

Eine weitere grundsätzliche Schwierigkeit bei allen existierenden oder zukünftigen Systemen zur Herstellung vollkeramischen Zahnersatzes aus gesinterten keramischen Werkstoffen hinsichtlich der Passgenauigkeit der fertigen Teile ist der keramische Schrumpf, also die mit dem verdichtenden Sinterprozess einhergehende Volumenschwindung keramischer Formteile. Dieser Sinterschrumpf lässt sich zwar innerhalb gewisser Grenzen reduzieren, aber nicht völlig vermeiden. Deshalb wird der mit dem Sinterschritt verbundene Sinterschrumpf beispielsweise indirekt dadurch vermieden, dass man bereits gesinterte Keramik (CADCAM-Verfahren, s. o.) verarbeitet oder versucht, auf andere Art und Weise ein porenfreies Feststoffgefüge zu erreichen (Glasinfiltration der weichen, porösen Keramik-Käppchen beim InCeram-Verfahren, s. o.). Auch bei der elektrophoretischen Abscheidung von Keramikpartikeln muss das erhaltene keramische Formteil anschließend gesintert werden, so dass sich auch hier das geschilderte Problem des Sinterschrumpfes zeigt.

Die Erfindung stellt sich deshalb die Aufgabe, dazu beizutragen, dass bei der Herstellung vollkeramischer Dentalformteile eine hohe Passgenauigkeit mit den Grundstrukturen, für die sie vorgesehen sind, erreicht wird. Dabei sollen insbesondere die nachteiligen Effekte des geschilderten Sinterschrumpfes vermieden werden.

Diese Aufgabe wird gelöst durch das Modellmaterial mit den Merkmalen des Anspruchs 1 und durch das Verfahren mit den Merkmalen des Anspruchs 5. Bevorzugte Ausführungsformen dieses Modellmaterials bzw. Verfahrens sind in den abhängigen Ansprüchen 2 bis 4 bzw. 6 bis 10 beschrieben. Der Wortlaut sämtlicher Ansprüche wird hiermit durch Bezugnahme zum Inhalt dieser Beschreibung gemacht.

Zum besseren Verständnis der Erfindung sei im folgenden kurz die Herstellung und Weiterverarbeitung zahntechnischer Modelle erläutert. Der Zahn oder die Zähne, die mit einem Dentalformteil, z. B. Krone, Brücke oder dergleichen, versehen werden sollen, werden vom Zahnarzt in bekannter Weise präpariert. Auch ein Implantataufbauteil kann als Ausgangspunkt dienen. Von dieser Mundsituation nimmt der Zahnarzt einen Abdruck mit Hilfe eines aushärtenden Elastomermaterials. Hier kann es sich beispielsweise um einen Silikonkunststoff handeln. Der so erhaltene Abdruck stellt ein Negativmodell der vom Zahnarzt vorgenommen Präparation dar. Dieser Abdruck, d. h. das Negativmodell wird dem Zahntechniker übergeben, der diesen Abdruck mit Hilfe eines geeigneten Modellmaterials, meist einem sogenannten Dentalgips, ausgießt. Nach dem Abbinden des Gipses entsteht ein Positivmodell, das sogenannte Meistermodell, welches der Präparation des Zahnarztes exakt entspricht. Dieses Meistermodell wird üblicherweise als Vorlage zurückbehalten. Es wird dazu verwendet, ein oder mehrere Arbeitsmodelle herzustellen, die dann weiterverarbeitet werden. Die Herstellung des Arbeitsmodells erfolgt durch Duplieren, d. h. mit Hilfe eines Dupliermaterials, beispielsweise Silikonkunststoff, wird ein Negativmodell hergestellt, das dann wiederum mit Dentalgips ausgegossen wird. Auf diese Weise wird ein weiteres Positivmodell, nämlich das Arbeitsmodell erstellt.

In Übereinstimmung mit dem soeben geschilderten Verfahrensablauf war es für den Fachmann bisher selbstverständlich, ein Modellmaterial mit einer möglichst geringen Expansion beim Abbinden bzw. Aushärten zu verwenden. Nur auf diese Weise kann nämlich die geforderte Dimensionstreue zwischen Präparation des Zahnarztes auf der einen Seite und Meistermodell bzw. Arbeitsmodell auf der anderen Seite gewährleistet werden. Deshalb sind beispielsweise die Abbindeexpansionen von gängigen Modellmaterialien wie Dentalgipsen in der Regel sehr gering. Diese Abbindeexpansion lässt sich nach den bekannten Beziehungen der Dilatometrie als lineare Ausdehnung ΔI/I₀ oder Volumenausdehnung ΔV/V₀ nach üblichen Methoden bestimmen. Die lineare Ausdehnung bei handelsüblichen Dentalgipsen, d. h. die Längenänderung, die ein entsprechender Gipskörper beim Abbinden erfährt, liegt bei weniger als 0,3 %. Grundsätzlich werden möglichst geringe Werte angestrebt. So liegen die linearen Expansionswerte bei den häufig gebrauchten Superhartgipsen der sogenannten Klasse IV bei ≤ 0,15 %. Solche Dentalgipse beschreibt auch die DE 3500515 A.

Das erfindungsgemäße Modellmaterial für Dentalzwecke zeichnet sich dagegen dadurch aus, dass es aus einem sogenannten Dentalgips besteht, der beim Abbinden bzw. Aushärten eine lineare Expansion von mindestens 0,5 %, vorzugsweise von mindestens 1 % aufweist. Bevorzugte Werte für die lineare Expansion beim Abbinden/Aushärten liegen zwischen 4 % und 12 %. Innerhalb dieses Bereichs sind wiederum Werte zwischen 8 % und 10 % hervorzuheben.

Ein Modellmaterial wie es die Erfindung bereitstellt, widerspricht dem bisherigen Verständnis des Fachmanns völlig. Wie bereits erläutert, war es bisher das Ziel, Modellmaterialien mit einer möglichst geringen Expansion beim Abbinden/Aushärten zur Verfügung zu stellen. Die Erfindung stellt nun bewusst auf Modellmaterialien mit höheren Expansionen ab, um auf diese Weise den bei der Herstellung von vollkeramischen Dentalformteilen auftretenden Sinterschrumpf zu kompensieren. Wird nämlich das Meistermodell oder bevorzugt das Arbeitsmodell bewusst "überdimensioniert", so kann der Sinterschrumpf in Kauf genommen werden. Sind das Expansionsverhalten des Modellmaterials und das Sinterschrumpfverhalten der Keramik bekannt, so kann ein exakt dimensioniertes vollkeramisches Dentalformteil zur Verfügung gestellt werden.

Bei der Erfindung besteht das Modellmaterial vollständig oder hauptsächlich aus Gips. In der Regel handelt es sich im vorliegenden Fall dann um sogenannte Dentalgipse, die den besonderen Anforderungen auf dem Dentalgebiet, beispielsweise bezüglich der Modellierfähigkeit und der sogenannten Zeichnungsgenauigkeit Rechnung tragen. Gips ist in der Summe seiner Eigenschaften für den Zahntechniker nach wie vor das Modellmaterial der Wahl. Bei exakter und produktgerechter Verarbeitung eignet sich Gips für alle Arten von Modellen in der Zahntechnik und deren Herstellung.

Feinpulvriger Dentalgips, chemisch CaSO₄ · ½ H₂O ("Calciumsulfat-Halbhydrat"), wird mit einer bestimmten Menge Wasser (H₂O) angemischt und zur Herstellung von Gipsduplikaten von Zähnen bzw. Gebissen verwendet. Der sich beim Anmischen bildende Gipsbrei wird in eine leicht entfernbare Form aus Dupliermaterial (meist Silikon) gefüllt, die dem Abdruck der Mundsituation entspricht. Die Masse bindet dann unter Reaktion mit dem Wasser zu CaSO₄ · 2 H₂O, dem Calciumsulfat-Dihydrat ab:

CaSO₄ · ½ H₂O + 1 ½ H₂O → CaSO₄ · 2 H₂O

Wie an der chemischen Formel abzulesen ist, wird ein Teil des zugegebenen Wassers beim Abbinden chemisch als sog. "Kristallwasser" gebunden. Beim Abbindevorgang verfestigt bzw. erhärtet der Gips. Es wird Wärme freigesetzt und der Prozess geht mit einer reproduzierbaren Expansion einher, die als lineare Ausdehnung ΔI/I oder als Volumenausdehnung ΔV/V bestimmbar ist. Diese Expansion ist bei den bisher bekannten Dentalgipsen niedrig und bei den erfindungsgemäßen Dentalgipsen bewusst hoch eingestellt.

Bei detaillierter Betrachtung ist der Abbindevorgang eine Summe von Einzelprozessen. Durch die Mischung des trockenen Gipspulvers mit Wasser entsteht eine übersättigte Lösung von Calciumsulfat-Halbhydrat, das Wasser aufnimmt und zu Dihydrat wird. Ausgehend von Kristallisationskeimen wachsen durch Aufnahme weiterer Dihydratmoleküle sog. Cluster, die weiter zu Kristallen wachsen. Durch die Bildung neuer Keime sowie das ständige Wachsen der Dihydrat-Kristalle entsteht so langsam ein immer fester werdendes Netzwerk von sich gegenseitig verhakenden und durchdringenden Kristallen, dessen Volumen größer ist als die Summe der einzelnen Kristallvolumina. Dies äußert sich makroskopisch dadurch, dass der Gips beim Abbinden die bereits erwähnte (Volumen)Expansion erfährt. Zusätzlich wird Energie in Form von Wärme frei.

Erfindungsgemäß sind im erfindungsgemäßen Modellmaterial Zusätze enthalten, die insbesondere auf den Abbinde- bzw. Aushärtevorgang Einfluss nehmen. Diese Zusätze beeinflussen Parameter wie die Expansion beim Abbinden/Aushärten, die Zeitdauer des Abbindens/Aushärtens die Härte des erhaltenen Modells und dergleichen. Erfindungsgemäß handelt es sich bei den Zusätzen um anorganische Substanzen. Dabei werden Silikate als Zusatz zur Erhöhung der Volumenexpansion verwendet. Solche Silikate können beispielsweise in Form von Kieselsol eingesetzt werden. Es ist dabei erfindungsgemäß möglich, die Silikate dem Gipspulver entweder direkt oder in Form silikathaltiger Anmischflüssigkeiten zuzugeben.

Das ebenfalls von der Erfindung umfasste Verfahren dient zur Herstellung eines zahntechnischen Modells, insbesondere eines zahntechnischen Meistermodells oder vorzugsweise Arbeitsmodells. Dieses Verfahren zeichnet sich dadurch aus, dass das Modell aus einem Modellmaterial mit einer erhöhten Expansion geformt wird und man dieses Modell anschließend abbinden oder aushärten lässt. Dabei wird das oben bereits beschriebene erfindungsgemäße Modellmaterial eingesetzt.

Die Expansion des Modells, die bei dem erfindungsgemäßen Verfahren erhalten wird, kann zusätzlich dadurch in gewünschter Weise erhöht werden, indem man das geformte Modell beim Abbinden/Aushärten über einen gewissen Zeitraum mindestens teilweise, vorzugsweise vollständig in eine Flüssigkeit, insbesondere ein Lösungsmittel eintaucht. Bei der Flüssigkeit handelt es sich vorzugsweise um die Flüssigkeit, mit der das Modellmaterial versetzt, insbesondere angerührt wird, um es in die für das Ausgießen der Form nötige breiige bzw. pastöse Form zu bringen. Im Falle der Verwendung von Dentalgips als Modellmaterial handelt es sich bei dieser Flüssigkeit üblicherweise um Wasser. In diesen Fällen lässt man das Gipsmaterial also dementsprechend unter Wasser abbinden.

Bei dem erfindungsgemäßen Verfahren ist es weiter bevorzugt, dass nach dem Abbinden/Aushärten erhaltene Modell mindestens teilweise zu trocknen. Dies geschieht üblicherweise durch einfaches Stehenlassen der Modelle an Luft, wobei üblicherweise ein Zeitraum zwischen 0,5 Stunden bis 3 Stunden ausreicht. Bei der Trocknung verdunstet das im Gips nicht als Kristallwasser chemisch gebundene Wasser. Der Trocknungsprozess kann durch Anwendung erhöhter Temperaturen unterstützt werden. Bei bevorzugten Ausführungsformen des erfindungsgemäßen Verfahrens wird zum Trocknen der Modelle mindestens ein Mikrowellentrocknungsschritt angewendet. Die Mikrowellentrocknung dauert in der Regel nur wenige Minuten und kann in einem haushaltsüblichen Mikrowellengerät vorgenommen werden.

Schließlich umfasst die Erfindung die Verwendung der bereits beschriebenen erfindungsgemäßen Modellmaterialien zur Herstellung vollkeramischer Dentalformteile. Dabei wird der beim Sintern der auf ein Meistermodell oder Arbeitsmodell aufgebrachten Dentalformteile eintretende Sinterschrumpf durch die bei der Herstellung des Meistermodells/Arbeitsmodells eintretende Volumenexpansion mindestens teilweise, vorzugsweise vollständig kompensiert. In diesem Zusammenhang wird auf die bisherige Beschreibung ausdrücklich Bezug genommen. Wie bereits beschrieben wird durch das erfindungsgemäße Modellmaterial, durch das erfindungsgemäße Verfahren und durch die erfindungsgemäße Verwendung sichergestellt, dass die vollkeramischen Dentalformteile exakt auf die vom Zahnarzt im Mund hergestellte Präparation passen.

Bei der Herstellung der vollkeramischen Dentalformteile wird dann vorzugsweise eine Suspension keramischer Partikel, der sogenannte keramische Schlicker, auf das Modell, üblicherweise ein Arbeitsmodell, aufgebracht. Dieses Arbeitsmodell ist aus dem erfindungsgemäßen Modellmaterial hergestellt und weist dementsprechend aufgrund der eingetretenen Volumenexpansion größere Abmessungen/Dimensionen auf als die vom Zahnarzt präparierte Grundstruktur im Mund. Dieses Arbeitsmodell besitzt dementsprechend üblicherweise auch größere Abmessungen/Dimensionen als das Meistermodell, das die Mundsituation exakt wiedergeben soll und zweckmäßigerweise nicht aus dem erfindungsgemäßen Modellmaterial hergestellt wird. Durch die größeren Abmessungen/Dimensionen des Arbeitsmodells, auf das der keramische Schlicker aufgebracht wird, wird der im Sinterschritt eintretende Sinterschrumpf bereits vorab berücksichtigt.

In diesem Zusammenhang sei erwähnt, dass das für das Aufbringen des keramischen Schlickers endgültig verwendete Arbeitsmodell erfindungsgemäß auch in mehreren Durchgängen hergestellt werden kann, je nachdem welches erfindungsgemäße Modellmaterial eingesetzt wird. Auf diese Weise kann man sich den gewünschten größeren Abmessungen des Arbeitsmodells zur Kompensation des Sinterschrumpfes sukzessive annähern oder gegebenenfalls sogar verschiedene vollkeramische Formteile erstellen und deren Passung mit dem Meistermodell testen.

Für den Fall, dass das vollkeramische Formteil im Mund unlösbar mit der dortigen Grundstruktur verbunden werden soll (nicht-herausnehmbarer Zahnersatz) kann bei der Herstellung des Modells (Arbeitsmodells) zusätzlich auch die Schichtdicke des Klebers oder Zahnzements, die beim Einbringen des Formteils in den Mund notwendig sind, berücksichtigt werden. Dies ist selbstverständlich im Falle von herausnehmbarem Zahnersatz nicht erforderlich.

Die keramische Suspension kann vorzugsweise durch elektrophoretische Abscheidung auf das Modell (Arbeitsmodell) aufgebracht werden. Die Grundlagen und die Durchführung einer solchen elektrophoretischen Abscheidung sind dem Fachmann bekannt. Dabei wird in Flüssigkeit dispergiertes, in diesem Fall keramisches Pulver, mit Hilfe eines elektrischen Feldes auf dem Modell als bereits vorverdichtete Schicht abgeschieden. Der auf diese Weise erhaltene keramische Körper, der sogenannte Grünkörper, wird, gegebenenfalls nach Trocknung und Entformung vom Modell, gesintert.

Bei der elektrophoretischen Formgebung wird das Modell der Mundsituation (Arbeitsmodell), das elektrisch, z. B. mit Leitsilberlack kontaktiert ist, als Elektrode in einen Stromkreis geschalten. Als Gegenelektrode dient beispielsweise eine Pt-Elektrode, deren Form je nach Form des Modells variiert werden kann, um ein hohes homogenes elektrisches Feld für das gesamte Modell zu erreichen.

Die Abscheidung des keramischen Schlickers auf das Arbeitsmodell erfolgt bei konstant gehaltener Spannung bzw. bei konstant gehaltenem Strom normalerweise über einen Zeitraum von 1 bis 60 Minuten. Typische Werte für die Abscheidespannung bzw. Abscheideströme liegen zwischen 1 und 100 V bzw. zwischen 0 und 500 mA. Die bei Verwendung der elektrophoretischen Abscheidung erhaltenen Gründichten sind üblicherweise größer als 70 %, vorzugsweise größer als 80 % der theoretischen Dichte. Die elektrophoretische Abscheidung kann gegebenenfalls automatisiert mit Hilfe eines entsprechenden Geräts erfolgen.

Die verwendeten Suspensionen keramischer Partikel sind Suspensionen dispergierter keramischer Pulver in geeigneten Lösungsmitteln. Wie erwähnt spricht man hier auch von sogenannten keramischen Schlickern. Als Lösungsmittel werden vorzugsweise polare Lösungsmittel verwendet, wobei es sich insbesondere um Wasser, Alkohole und deren Mischungen, oder Mischungen aus Wasser mit Alkoholen handelt. Vorzugsweise werden polare Lösungsmittel mit Dielektrizitätszahlen im Bereich zwischen 15 und 85, vorzugsweise im Bereich von 15 bis 20 verwendet.

Bei den keramischen Partikeln handelt es sich vorzugsweise um oxidkeramische Partikel, insbesondere um Aluminiumoxid (Al₂O₃)-Partikel und/oder Zirkonoxid (ZrO₂)-Partikel, oder deren Mischungen. Die Korngrößen der keramischen Partikel liegen vorzugsweise zwischen 1 nm und 100 µm, vorzugsweise zwischen 100 nm und 10 µm. Insbesondere sind die keramischen Partikel in der Suspension in einer Menge zwischen 10 und 90 Gewichtsprozent, vorzugsweise zwischen 40 und 60 Gewichtsprozent, bezogen auf das Gesamtgewicht der Suspension, enthalten.

Bei weiteren Ausführungsformen können innerhalb der Suspension mindestens 2 Fraktionen keramischer Partikel mit unterschiedlicher mittlerer Korngröße enthalten sein. Auf diese Weise kann erreicht werden, dass die Dichte des abgeschiedenen Grünkörpers erhöht wird, da die keramischen Partikel mit kleinerer mittlerer Korngröße die Zwischenräume zwischen den keramischen Partikeln mit größerer mittlerer Korngröße zumindest teilweise auffüllen. Bekanntermaßen folgt die Korngrößenverteilung einer Fraktion keramischer Partikel mit bestimmter mittlerer Korngröße einer Gauß-Verteilung. Dementsprechend sind bei den beschriebenen Ausführungen (um in diesem Bild zu bleiben) die zwei oder mehr Gauß-Kurven gegeneinander verschoben.

Üblicherweise sind noch Bindemittel Bestandteil der Suspension, wobei es sich vorzugsweise um mindestens einen Polyvinylalkohol oder um mindestens ein Polyvinylbutyral handelt. Solche Bindemittel dienen u. a. zur Verbesserung sowohl des Trocknungsverhaltens als auch der Festigkeiten der resultierenden Grünkörper. Die Bindemittel sind in der Suspension, bezogen auf deren Feststoffgehalt, vorzugsweise in Mengen zwischen 0,1 und 20 Gewichtsprozent, insbesondere zwischen 0,2 und 10 Gewichtsprozent enthalten.

Die verwendeten Schlicker zeichnen sich durch Viskositäten im Bereich von 1 mPa*s bis 50 mPa*s, vorzugsweise im Bereich von 3 bis 10 mPa*s bei einer Scherrate von 600 s⁻¹ aus. Die durch die zugegebene Dispergierhilfe erhaltenen Zeta-Potentiale der Schlicker liegen zwischen ± 1 mV und ± 100 mV, vorzugsweise zwischen ± 30 mV und ± 50 mV.

Der auf diese Weise hergestellte Grünkörper weist vorzugsweise eine durchschnittliche Schichtdicke von 0,2 bis 2 mm, insbesondere von 0,8 bis 1,2 mm auf. Dadurch können nach dem Sinterschritt die erwünschten Schichtdicken des vollkeramischen Formteils bereitgestellt werden.

Der keramische Grünkörper wird bei den Temperaturen gesintert, die sich aus den verwendeten Keramikmaterialien ergeben. Vorzugsweise liegt die Sintertemperatur zwischen 1100 °C und 1700 °C, insbesondere zwischen 1150 °C und 1300 °C. Vorzugsweise beträgt die Sintertemperatur ca. 1200 °C.

Die Sinterzeit wird ebenfalls z. B. in Abhängigkeit von dem verwendeten Keramikmaterial gewählt. Hier sind bevorzugte Sinterzeiten zwischen 2 und 10 Stunden, insbesondere zwischen 4 und 6 Stunden zu nennen. Bei weiteren bevorzugten Ausführungsformen wird ca. 5 Stunden gesintert.

Um eine homogene Temperaturverteilung im Grünkörper zu erreichen, wird dieser allmählich auf die endgültige Sintertemperatur gebracht. Bevorzugte Aufheizraten betragen hier zwischen 1 und 20 °C/min, insbesondere zwischen 5 und 10 °C/min. Innerhalb des zuletzt genannten Bereichs sind Aufheizraten zwischen 5 und 7,5 °C/min weiter bevorzugt.

Vorzugsweise wird im Sinterschritt so vorgegangen, dass das Arbeitsmodell zusammen mit dem darauf abgeschiedenen Grünkörper bei Raumtemperatur an Luft getrocknet und dann anschließend in den Ofen überführt wird. Dort wird das Arbeitsmodell zusammen mit dem Grünkörper bis auf ca. 900 °C erhitzt, wobei hier eine vergleichsweise geringe Aufheizrate verwendet werden kann. Dieses Aufheizen kann stufenweise erfolgen, wobei Haltezeiten bei den entsprechenden Temperaturen vorgesehen sein können. Durch dieses Erhitzen wird der Grünkörper vorgesintert, wobei das Gipsmaterial des Arbeitsmaterials schrumpft, da der Gips sein Kristallwasser teilweise verliert. Dann wird das Arbeitsmodell zusammen mit dem Grünkörper kurz aus dem Ofen genommen und der Grünkörper vom Arbeitsmodell entformt. Dies geschieht leicht, da das Arbeitsmodell wie beschrieben geschrumpft ist. Dann wird der vorgesinterte Grünkörper, beispielsweise in Form eines Käppchens wieder in den Ofen gegeben. Dann wird der Ofen, vorzugsweise mit einer vergleichsweise hohen Aufheizrate auf die endgültige Sintertemperatur gebracht und das Formteil fertig gesintert.

Nach dem Sinterschritt werden vollkeramische Formteile mit Dichten von mehr als 90 % der theoretischen Dichte, vorzugsweise mehr als 95 % der theoretischen Dichte erhalten. Solche Vollkeramikteile, beispielsweise in Form eines Käppchens, können dann in üblicher Weise wie ein Metallkäppchen mit Verblendkeramik versehen und gebrannt werden. Auf diese Weise entsteht der endgültige Zahnersatz, der beispielsweise in Form einer Krone oder Brücke in den Mund des Patienten eingesetzt wird. Selbstverständlich kann der so herstellbare Zahnersatz auch auf dentale Suprakonstruktionen, wie beispielsweise Implantatteile aufgesetzt werden.

Weitere Merkmale der Erfindung ergeben sich aus den nachfolgenden Beispielen in Verbindung mit den Unteransprüchen. Hierbei können die einzelnen Merkmale jeweils für sich allein oder zu mehreren in Kombination miteinander verwirklicht sein.

### Beispiele

### Beispiel 1:

Anhand eines handelsüblichen Dentalgipses soll zunächst gezeigt werden, dass sich die Volumenexpansion derartiger Modellmaterialien durch geeignete Maßnahmen erhöhen lässt. Für die im folgenden beschriebenen Versuche wird dabei ein handelsüblicher Dentalgips der sogenannten Klasse II, nämlich der Dentalgips mit der Bezeichnung "Alamo" der Firma Hinrichs, Deutschland verwendet. Nach Herstellerangaben besitzt dieser Dentalgips eine lineare Abbindeexpansion von 0,29 %. Es wird ein Anmischverhältnis von 100 g Gipspulver auf 50 ml Wasser angegeben.

### Beispiel 1a: Wirkung silikatischer Zusätze.

100 g des oben genannte Gipspulvers werden mit 50 ml einer 10 %-igen Kieselsol-Lösung (Art Nr. 33,844-3 der Aldrich Chemicals) gemischt und anschließend 30 Sekunden lang unter Vakuum gerührt. Der so erhaltene Gipsbrei wird auf einem Rüttler in Duplierformen aus Silikon eingefüllt. Die Duplierformen sind dabei der Präparation eines Zahnstumpfes nachgebildet. Nach einer Abbindezeit von 25 Minuten wird das Gipsmodell aus der Duplierform entformt und eine Stunde lang an Luft getrocknet. Die anschließende Messung der linearen Abbindeexpansion (Längenänderung nach dem Abbinden) beträgt 0,5 %.

### Beispiel 1b: Wirkung der Abbindung unter Wasser.

100 g des Gipspulvers und 50 ml Wasser werden gemischt und anschließend 30 Sekunden unter Vakuum gerührt. Der so erhaltene Gipsbrei wird auf einem Rüttler in Duplierformen aus Silikon eingefüllt. Nach einer Abbindezeit von 20 Minuten wird das Gipsmodell aus der Duplierform entformt. Anschließend lässt man das Gipsmodell weitere 60 Minuten unter Wasser abbinden. Die lineare Abbindexpansion beträgt 0,6 %.

### Beispiel 1c: Wirkung von silikatischen Zusätzen und Abbindung unter Wasser.

100 g des Gipspulvers und 50 ml einer 10 %-igen Kieselsol-Lösung (Art Nr. 33,844-3 der Aldrich Chemicals) werden gemischt und anschließend 30 Sekunden lang unter Vakuum gerührt. Der so erhaltene Gipsbrei wird auf einem Rüttler in Duplierformen aus Silikon eingefüllt. Nach einer Abbindezeit von 20 Minuten wird das Gipsmodell aus der Duplierform entformt. Dieses Gipsmodell lässt man anschließend 60 Minuten unter Wasser abbinden. Dann folgt ein Trocknungsschritt von 1 Stunde an Luft. Die lineare Abbindeexpansion des erhaltenen Gipsmodells beträgt 0,7 %.

### Beispiel 2:

Ein handelsüblicher Dentalgips der Klasse III, Marke Heraeus, Typ "Moldano", Farbe hellblau, hat nach Herstellerangaben eine Abbindeexpansion gemäß EN ISO 6873 von linear 0,16 % nach 40 Minuten Abbindezeit.

Am Beispiel der Verarbeitung dieses Gipses wurde gegenüber dem Standardvorgehen (1) die expansionserhöhende Wirkung von Abbindung unter Wasser (2), silikatischen Zusätzen zum Gips (3), zusätzlich einer silikatischen Anmischflüssigkeit (4) sowie aller drei Maßnahmen zusammen (5) nachvollzogen:
(1) 100 g Gipspulver und 30 ml Wasser werden manuell gemischt, anschließend 40 Sekunden unter Vakuum gerührt, unter leichtem Rütteln in ein Extensometer gefüllt und anschließend gemäß EN ISO 6873 die Abbindeexpansion gemessen. Nach 40 Minuten Abbindezeit ergibt sich eine lineare Abbindeexpansion von 0,16 %.
(2) 100 g Gipspulver und 30 ml Wasser werden manuell gemischt, anschließend 40 Sekunden unter Vakuum gerührt, unter leichtem Rütteln in ein Extensometer gefüllt und anschließend gemäß EN ISO 6873 die Abbindeexpansion gemessen, wobei die Gipsmasse ständig mit Wasser beträufelt und somit nass gehalten wurde. Nach 40 Minuten Abbindezeit ergibt sich eine lineare Abbindeexpansion von 0,31 %.
(3) Ein Gemisch aus 90 g Gipspulver, 10 g Montmorillonit-Schichtsilikat (Montmorillonit K-10, Aldrich Chemicals) und 40 ml Wasser werden manuell gemischt, anschließend 40 Sekunden unter Vakuum gerührt, unter leichtem Rütteln in ein Extensometer gefüllt und anschließend gemäß EN ISO 6873 die Abbindeexpansion gemessen. Nach 40 Minuten Abbindezeit ergibt sich eine lineare Abbindeexpansion von 0,38 %.
(4) Ein Gemisch aus 90 g Gipspulver, 10 g Montmorillonit-Schichtsilikat (Montmorillonit K-10, Aldrich Chemicals), 30 ml Wasser und 10 ml Kieselsol werden manuell gemischt, anschließend 40 Sekunden unter Vakuum gerührt, unter leichtem Rütteln in ein Extensometer gefüllt und anschließend gemäß EN ISO 6873 die Abbindeexpansion gemessen. Nach ca. 30 Minuten Abbindezeit ergibt sich eine lineare Abbindeexpansion von 0,61 %.
(5) Ein Gemisch aus 90 g Gipspulver, 10 g Montmorillonit-Schichtsilikat (Montmorillonit K-10, Aldrich Chemicals), 30 ml Wasser und 10 ml Kieselsol werden manuell gemischt, anschließend 40 Sekunden unter Vakuum gerührt, unter leichtem Rütteln in ein Extensometer gefüllt und anschließend gemäß EN ISO 6873 die Abbindeexpansion gemessen, wobei die Gipsmasse ständig mit Wasser beträufelt und somit nass gehalten wurde. Nach ca. 30 Minuten Abbindezeit ergibt sich eine lineare Abbindeexpansion von 1,06%.

## Patentansprüche

1. Modellmaterial für zahntechnische Zwecke, **dadurch gekennzeichnet, dass** es aus einem sogenannten Dentalgips besteht, der beim Abbinden bzw. Aushärten eine lineare Expansion von mindestens 0,5 % aufweist und einen anorganischen Zusatz enthält, wobei es sich bei dem anorganischen Zusatz um mindestens ein Silikat und/oder ein Kieselsol handelt.

2. Modellmaterial nach Anspruch 1, **dadurch gekennzeichnet, dass** der Dentalgips eine lineare Expansion von mindestens 1 % aufweist.

3. Modellmaterial nach Anspruch 2, **dadurch gekennzeichnet, dass** der Dentalgips eine lineare Expansion von 4 % bis 12 % aufweist.

4. Modellmaterial nach Anspruch 3, **dadurch gekennzeichnet, dass** der Dentalgips eine lineare Expansion von 8 % bis 10 % aufweist.

5. Verfahren zur Herstellung eines zahntechnischen Modells, insbesondere eines zahntechnischen Arbeitsmodells, **dadurch gekennzeichnet, dass** das Modell aus einem sogenannten Dentalgips, der beim Abbinden bzw. Aushärten eine lineare Expansion von mindestens 0,5 % aufweist und einen anorganischen Zusatz enthält, wobei es sich bei dem anorganischen Zusatz um mindestens ein Silikat und/oder ein Kieselsol handelt, geformt wird und man das so erhaltene Modell anschließend abbinden lässt oder aushärtet.

6. Verfahren nach Anspruch 5, **dadurch gekennzeichnet, dass** das Modell aus einem Modellmaterial, wie es in den Ansprüchen 2 bis 4 definiert ist, geformt wird.

7. Verfahren nach Anspruch 5 oder 6, **dadurch gekennzeichnet, dass** der Abbinde- bzw. Aushärteschritt mindestens teilweise, vorzugsweise vollständig unter mindestens einer Flüssigkeit, vorzugsweise unter Wasser durchgeführt wird.

8. Verfahren nach einem der Ansprüche 5 bis 7, **dadurch gekennzeichnet, dass** das Modell nach dem Abbinde- bzw. Aushärteschritt getrocknet wird.

9. Verfahren nach Anspruch 8, **dadurch gekennzeichnet, dass** das Trocknen durch Stehenlassen an Luft, vorzugsweise über einen Zeitraum von 30 Minuten bis 3 Stunden, erfolgt.

10. Verfahren nach Anspruch 8, **dadurch gekennzeichnet, dass** das Trocknen mit Hilfe von Mikrowellen erfolgt.

11. Verwendung von sogenanntem Dentalgips, der beim Abbinden bzw. Aushärten eine lineare Expansion von mindestens 0,5 % aufweist und einen anorganischen Zusatz enthält, wobei es sich bei dem anorganischen Zusatz um mindestens ein Silikat und/oder ein Kieselsol handelt, zur Herstellung vollkeramischer Dentalformteile, wobei der Sinterschrumpf, der beim Sintern eines auf einem Arbeitsmodell gebildeten keramischen Grünkörpers eintritt, durch die Expansion des Modellmaterials bei der Herstellung des Arbeitsmodells mindestens teilweise kompensiert wird.

12. Verwendung nach Anspruch 11, **dadurch gekennzeichnet, dass** der Dentalgips eine lineare Expansion von mindestens 1 % aufweist.

13. Verwendung nach Anspruch 11 oder 12, **dadurch gekennzeichnet, dass** der Sinterschrumpf vollständig kompensiert wird.

## Claims

1. Model material for dental purposes, **characterized in that** it consists of dental plaster which has a linear expansion on setting or curing of at least 0.5% and contains an inorganic additive, wherein the inorganic additive is at least one silicate and/or silica sol.

2. Model material according to Claim 1, **characterized in that** the dental plaster has a linear expansion of at least 1%.

3. Model material according to Claim 2, **characterized in that** the dental plaster has a linear expansion of from 4% to 12%.

4. Model material according to Claim 3, **characterized in that** the dental plaster has a linear expansion of from 8% to 10%.

5. Process for producing a dental model, in particular a dental working model, **characterized in that** the model is shaped from dental plaster which has a linear expansion on setting or curing of at least 0.5% and contains an inorganic additive, wherein the inorganic additive is at least one silicate and/or a silica sol, and the model obtained in this way is subsequently allowed to set or is cured.

6. Process according to Claim 5, **characterized in that** the model is shaped from a model material as defined in any of Claims 2 to 4.

7. Process according to Claim 5 or 6 **characterized in that** the setting or curing step is at least partly, preferably entirely, carried out under at least one liquid, preferably under water.

8. Process according to any of Claims 5 to 7, **characterized in that** the model is dried after the setting or curing step.

9. Process according to Claim 8, **characterized in that** drying is carried out by allowing to stand in air, preferably for a period of from 30 minutes to 3 hours.

10. Process according to Claim 8, **characterized in that** drying is carried out with the aid of microwaves.

11. Use of dental plaster which has a linear expansion on setting or curing of at least 0.5% and contains an inorganic additive, wherein the inorganic additive is at least one silicate and/or a silica sol for producing full-ceramic shaped dental parts, where the sintering shrinkage which occurs on sintering a green ceramic body formed on a working model is compensated for at least partly by the expansion of the model material in the production of the working model.

12. Use according to Claim 11, **characterized in that** the dental plaster has a linear expansion of at least 1%.

13. Use according to Claim 11 or 12, **characterized in that** the sintering shrinkage is compensated for fully.

## Revendications

1. Matériau de moulage pour applications techniques dentaires, **caractérisé en ce qu'**il est constitué d'un plâtre dit dentaire qui, lors de sa prise et de son durcissement, présente une dilatation linéaire d'au moins 0,5 % et qui contient un additif inorganique, l'additif inorganique étant au moins un silicate et/ou un gel de silice.

2. Matériau de moulage selon la revendication 1, **caractérisé en ce que** le plâtre dentaire a une dilatation linéaire d'au moins 1 %.

3. Matériau de moulage selon la revendication 2, **caractérisé en ce que** le plâtre dentaire a une dilatation linéaire de 4 % à 12 %.

4. Matériau de moulage selon la revendication 3, **caractérisé en ce que** le plâtre dentaire a une dilatation linéaire de 8 % à 10 %.

5. Procédé de réalisation d'un moulage en technique dentaire, et en particulier d'un moulage de travail en technique dentaire, **caractérisé en ce que** le moulage est constitué d'un plâtre dit dentaire qui, lors de sa prise et de son durcissement, présente une dilatation linéaire d'au moins 0,5 % et qui contient un additif inorganique, l'additif inorganique étant au moins un silicate et/ou un gel de silice, et **en ce qu'**on laisse ensuite le moulage ainsi obtenu prendre ou durcir.

6. Procédé selon la revendication 5, **caractérisé en ce que** le moulage est constitué d'un matériau de moulage selon l'une des revendications 2 à 4.

7. Procédé selon les revendications 5 ou 6, **caractérisé en ce que** l'étape de prise ou de durcissement est réalisée au moins en partie et de préférence complètement dans au moins un liquide et de préférence dans l'eau.

8. Procédé selon l'une des revendications 5 à 7, **caractérisé en ce que** le moulage est séché après l'étape de prise et de durcissement.

9. Procédé selon la revendication 8, **caractérisé en ce que** le séchage s'effectue en laissant reposer à l'air, de préférence pendant une durée de 30 minutes à 3 heures.

10. Procédé selon la revendication 8, **caractérisé en ce que** le séchage s'effectue à l'aide de micro-ondes.

11. Utilisation d'un plâtre dit dentaire qui, lors de la prise et de son durcissement, présente une dilatation linéaire d'au moins 0,5 % et contient un additif minéral, l'additif minéral étant au moins un silicate et/ou un gel de silice, pour réaliser des pièces moulées dentaires entièrement en céramique, et dans lequel le retrait de frittage qui a lieu lors du frittage d'un corps cru en céramique formé sur un moulage de travail est compensé au moins en partie par la dilatation du matériau de moulage lors de la réalisation du moulage de travail.

12. Utilisation selon la revendication 11, **caractérisée en ce que** le plâtre dentaire a une dilatation linéaire d'au moins 1 %.

13. Utilisation selon les revendications 11 ou 12, **caractérisée en ce que** le retrait de frittage est complètement compensé.
